# EUROPEAN PATENT APPLICATION

(11) **EP 3 811 846 A1**
(43) Date of publication of application: **28.04.2021**
(21) Application number: 19823321.5
(22) Date of filing: 19.06.2019
(51) Int. Cl.: A61B 1/045, A61B 1/06, A61B 1/12

(54) **CAPSULE ROBOT HAVING SYMMETRICAL SIDE FORCE SENSING FUNCTION**

(30) Priority: 20.06.2018 CN 201810637247
(71) Applicant: Beijing Institute Of Technology, Beijing 100081 (CN); Huang, Qiang, Beijing 100081 (CN); Li, Jing, Beijing 100081 (CN); Zhou, Long, Beijing 100081 (CN); Paolo, Dario, Beijing 100081 (CN); Gastone, Ciuti, Beijing 100081 (CN); Zhang, Wenhui, Beijing 100081 (CN)
(72) Inventor: HUANG, Qiang, Beijing 100081 (CN); LI, Jing, Beijing 100081 (CN); ZHOU, Long, Beijing 100081 (CN); PAOLO, Dario, Beijing 100081 (CN); GASTONE, Ciuti, Beijing 100081 (CN); ZHANG, Wenhui, Beijing 100081 (CN)
(74) Representative: Vidon Brevets & Stratégie
(86) International application number: PCT/CN2019/000128
(87) International publication number: WO 2019/242293

(57) **Abstract**

The present invention discloses an endoscopic capsule-like robot having a symmetrical lateral force induction function, which can sense a multilateral interactive contact force between a capsule type head and an external environment, and includes: a capsule type head, a connection body, a force induction module arranged in the capsule type head, and a channel module used for connecting the capsule type head with the connection body. The force induction unit includes two or more thin-film type pressure sensors uniformly distributed along a circumferential direction of a head shell; the pressure sensors are used for sensing pressure data of the external environment on the capsule type head, transmitting the sensed pressure data to a force data output unit, and transmitting the pressure data to an external upper computer in a wireless manner. The capsule type head moves under the driving of an external single magnetic field or multiple magnetic fields, so as to detect an interactive contact force between the inner wall of a smaller lumen of the stomach and intestine or a small intestinal wall and the capsule type head. Channel tubes, entering a human body, of the capsule-like robot are flexible tubes, except the capsule type head, so that no pain is caused; and in addition, movement control is carried out through the external magnetic field, so that painless and all-around control observation can be realized

## Description

### TECHNICAL FIELD

The present invention relates to a capsule-like robot, and in particular, to a capsule-like robot having a symmetrical lateral force induction function, and pertains to the technical field of medical instruments.

### BACKGROUND

An endoscopic technology can effectively prevent and treat gastrointestinal diseases. Traditional endoscopes currently used in the market easily cause pain due to high rigidity of their insertion portions. A capsule-like robot is a new endoscopic technology, which can effectively reduce the degree of pain, and is an important trend of the endoscopic technology.

At the present, a passive capsule that has been promoted and applied can only transmit images, and cannot perform biopsy sampling and minimal surgical operations. An active capsule-like robot, different from the passive capsule, can move autonomously under the traction of an external robot, and can complete biopsy sampling and minimal surgical operations at the same time. During the movement of the active capsule-like robot, when multiple sides are in symmetrical contact and interaction with the gastrointestinal wall, information of a contact interaction force in each direction is obtained, and is very important for controlling the capsule-like robot to move along a specified path in the gastrointestinal lumen. After relevant literature is consulted, it is found that there is no active capsule-like robot integrated with a force induction function.

### SUMMARY

In view of this, the present invention provides a capsule-like robot having a symmetrical lateral force induction function. The capsule-like robot is provided with a force induction module, and sensors in the force induction module are symmetrically distributed on a shell of a capsule type head, and are used for monitoring a contact state under a condition of multilateral symmetric contact between the capsule-like robot and the gastrointestinal luminal wall.

The capsule-like robot having the symmetrical lateral force induction function includes: a capsule type head, a connection body, a force induction module arranged in the capsule type head, and a channel module used for connecting the capsule type head with the connection body.

The capsule type head includes: a head shell, a magnet mounted inside the head shell, and a camera module integrated with a light source; the magnet performs location and motion control on the capsule type head under the driving of an external magnetic field; a magnet mounting slot, a tool channel hole and a camera cable channel hole are machined in the head shell; an end, opposite to an end connected with the channel module, of the head shell is provided with a mounting slot which communicates with the camera cable channel hole and is used for mounting the camera module.

The force induction module is used for sensing pressure data of each contact side under a condition that the capsule-like robot is in multilateral symmetrical contact with an external environment; the force induction module includes: a force induction unit and a force data output unit; the force induction unit includes two or more thin-film type pressure sensors uniformly distributed in a spacing manner along a circumferential direction of the head shell; the pressure sensors are used for sensing pressure data of the external environment on a mounting side of the capsule type head, and transmitting the sensed pressure data to the force data output unit; and the force data output unit performs signal conditioning and analog-to-digital conversion on the received pressure data, and transmits the data to an external upper computer in a wireless manner.

The channel module includes: an external sealing tube, and a camera cable and a tool channel tube which are wrapped in the external sealing tube.

A tool channel interface and a camera control and power interface are arranged on the connection body; the tool channel interface communicates with the tool channel tube in the channel module; one end of the camera cable is connected with the camera module through the camera cable channel inside the head shell, and the other end of the camera cable is connected with an external power supply and a camera control device through the camera control and power interface on the connection body.

The channel module also includes a water/air channel tube wrapped in the external sealing tube; a water/air channel hole is machined inside the head shell; the end, opposite to the channel module connection end, of the head shell is provided with a water/air nozzle communicating with the water/air channel tube; a water supply equipment interface and an air supply equipment interface are arranged on the connection body; one end of the water/air channel tube communicates with the water/air nozzle through the water/air channel hole, and the other end of the water/air channel tube communicates with the water supply equipment interface and the air supply equipment interface.

After obtaining the pressure data of the external environment on each contact side of the capsule type head, the upper computer takes the pressure data as a contact interaction force between the external environment and the capsule type head to perform safety monitoring on the capsule-like robot, specifically as follows: a safety threshold of the contact interaction force of each contact side is set in the upper computer; and when the pressure data sensed by the pressure sensor of a certain contact side exceeds the corresponding safety threshold, the capsule-like robot is controlled to stop moving or a distance between the external magnetic field and the capsule type head is increased.

Beneficial effects:
(1) The capsule-like robot provided with the force induction module having the symmetrical lateral force induction function is applicable to monitoring the contact state under a condition of multilateral symmetric contact between the capsule-like robot and the gastrointestinal luminal wall during inspection for a small-diameter lumen in a gastrointestinal region, and senses a contact force phenomenon of the capsule-like robot and the gastrointestinal luminal wall through the force induction module, so that a deviation between the current posture of the capsule and the specified path can be determined, thereby realizing path planning based on force data information; and motion control can be better performed, and the safety can be better ensured.
(2) At the same time, the capsule-like robot is applicable, when a plurality of driving magnetic fields are symmetrically distributed around the capsule-like robot, to path tracking and control under the condition of multilateral symmetric contact between the capsule-like robot and an inspection environment where the capsule-like robot is located.

### BRIEF DESCRIPTION OF THE DRAWINGS

Fig. 1 is a schematic structural diagram of Embodiment 1;
Fig. 2 is a schematic structural diagram of a capsule type head in Embodiment 1;
Fig. 3 is a structural composition diagram of a head shell in Embodiment 1;
Fig. 4 is a cutaway view of a capsule type head in Embodiment 1;
Fig. 5 is a schematic structural diagram of a force data output unit in Embodiment 1;
Fig. 6 is a schematic structural diagram of a capsule type head in Embodiment 2;
Fig. 7 illustrates a structural composition diagram of a head shell in Embodiment 2 and a structural diagram of a partition plate;
Fig. 8 is a cutaway view of a capsule type head in Embodiment 2;
Fig. 9 is a schematic structural diagram of a force data output unit in Embodiment 2;
Fig. 10 is a schematic structural diagram of a capsule type head in Embodiment 3;
Fig. 11 is a structural composition diagram of a head shell in Embodiment 3;
Fig. 12 is a cutaway view of a capsule type head in Embodiment 3;
Fig. 13 is a schematic structural diagram of a force data output unit in Embodiment 3;
Fig. 14 is a schematic structural diagram of a capsule type head in Embodiment 4;
Fig. 15 illustrates a structural composition diagram of a head shell in Embodiment 4 and a structural diagram of a partition plate;
Fig. 16 is a cutaway view of a capsule type head in Embodiment 4; and
Fig. 17 is a schematic structural diagram of a force data output unit in Embodiment 4.

### DETAILED DESCRIPTION

The present invention is further described below in combination with the accompanying drawings and embodiments.

### Embodiment 1

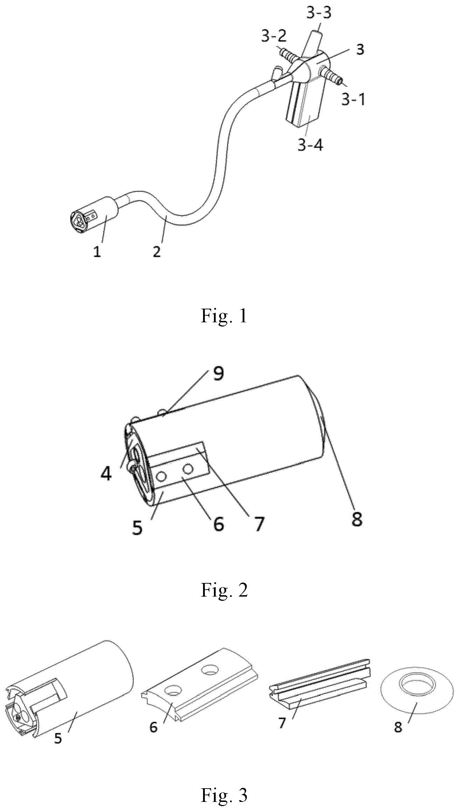

The present embodiment provides a capsule-like robot having a symmetrical lateral force induction function, referring to Fig. 1, including: a capsule type head 1, a force induction module, a channel module 2, and a connection body 3, wherein the force induction module is mounted in the capsule type head 1; one end of the channel module 2 is connected with the capsule type head 1, and the other end is connected with the connection body 3.

The channel module 2 includes: an external sealing tube, and a camera cable 11, a tool channel tube 13 and a water/air channel tube 15 which are wrapped inside the external sealing tube. The above-mentioned components are all made of a medical high polymer material that is nontoxic and harmless to a human body and cannot release toxic substances or gas, and are all flexible tubes.

Referring to Figs. 2 to 4, the capsule type head 1 includes: a head shell 5, a head end cover A4 and a head end cover B8 which are arranged at two ends of the head shell 5, and a circular-ring-shaped magnet 10 and a camera unit 12 which are mounted inside the head shell 5. An end, provided with the head end cover B8, of the head shell 5 is connected with the channel module 2. The head shell 5 is a cylindrical shell, and an end, connected with the head end cover B8, in the head shell is provided with a cylindrical cavity used for mounting the circular-ring-shaped magnet 10; and an axial line of the cylindrical cavity is superposed with an axial line of the cylindrical shell. An end, away from the channel module 2, of the head shell 5 is provided with a camera unit mounting hole communicating with a camera cable channel hole, and a water/air nozzle communicating with a water/air channel hole; the water/air channel hole, the tool channel hole and the camera cable channel hole are machined inside the head shell 5; in order to reduce the volume of the capsule type head 1 as much as possible, the water/air channel hole, the tool channel hole and the camera cable channel hole all fall within an envelope area for a center hole for mounting the circular-ring-shaped magnet 10, that is, the water/air channel hole, the tool channel hole and the camera cable channel hole run through the center hole of the circular-ring-shaped magnet 10. Therefore, after passing through the center hole of the circular-ring-shaped magnet 10 and the water/air channel hole in sequence, the water/air channel tube 15 communicates with the water/air nozzle. After passing through the center hole of the circular-ring-shaped magnet 10 and the camera cable channel hole in sequence, the camera cable 11 is connected with the camera unit 12 mounted in the camera unit mounting hole. The camera unit 12 has a waterproofing function to ensure that no fault occurs in a process of entering and leaving a human body. Furthermore, a light source used for illumination is integrated in the camera unit 12 to ensure that an endoscope can work normally in a dark environment.

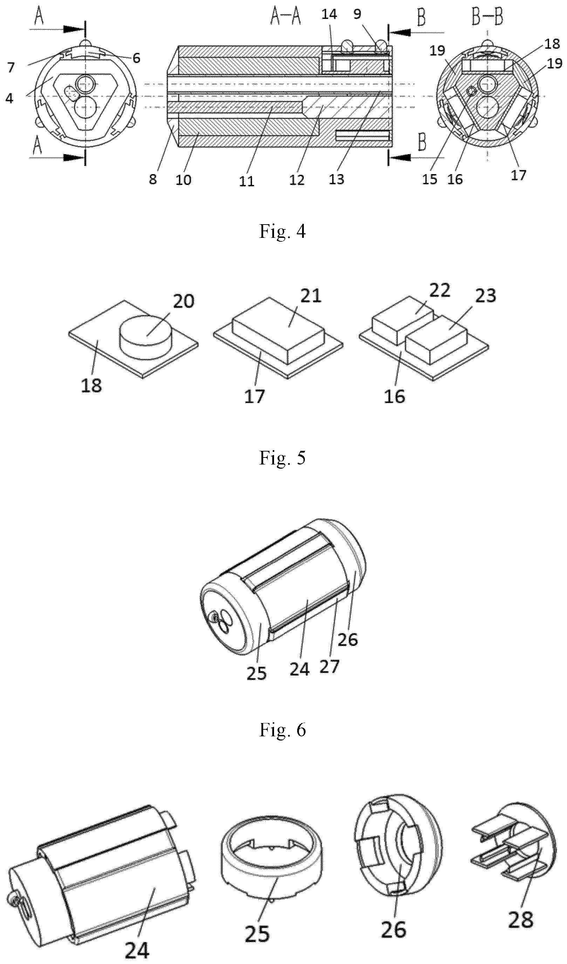

Referring to Figs. 4 to 5, the force induction module includes: a force induction unit and a force data output unit. The force induction unit includes six sensor feeler levers 9 and three thin-film type pressure sensors 14. Each pressure sensor 14 corresponds to two sensor feeler levers 9, and the sensor feeler levers 9 are used for sensing a pressure and transmitting the sensed pressure to the pressure sensor 14. The force data output unit includes three circuit boards. The three circuit boards are respectively a circuit board A16, a circuit board B 17 and a circuit board C18. The three circuit boards are uniformly distributed in a spacing manner along a circumferential direction of the head shell 5, so as to reduce the occupied volume as much as possible. The circuit board C18 is integrated with a battery 20, the circuit board B17 is integrated with a wireless transmission module 21, and the circuit board A16 is integrated with an operational amplifier circuit 22 and an analog-to-digital conversion module 23. The circuit board C18 is connected with the circuit board A16 and the circuit board B17 respectively through a cable 19. The cable 19 includes a power supply cable and a data transmission cable. The circuit board C18 supplies power to electronic components on the circuit board A16 and the circuit board B17 through the power supply cable, and mutual data transmission can be realized between two connected circuit boards through the data transmission cable. The three pressure sensors 14 are connected with the three circuit boards respectively in a one-to-one correspondence manner, so that after signals collected by the three pressure sensors 14 are processed by the operational amplifier circuit 22 and the analog-to-digital conversion module 23, the signals are finally transmitted in a wireless transmission manner via the wireless transmission module 21 to external control equipment.

A water supply equipment interface 3-1, an air supply equipment interface 3-2, a tool channel interface 3-3 and a camera control and power interface 3-4 are arranged in the connection body 3; the water supply equipment interface and the air supply equipment interface respectively communicate with the water/air channel tube 15 of the channel module 2; the tool channel interface communicates with the tool channel tube 13 of the channel module 2; and the camera control and power interface is used for allowing the camera cable 11 in the channel module 2 to pass, so as to be connected with an external camera control device.

A whole connection relationship of the capsule-like robot is as follows:
Referring to Figs. 2 to 5, the circular-ring-shaped magnet 10 is mounted in a cylindrical cavity of the head shell 5, and moves under the driving of an external magnetic field to drive the capsule type head 1 to move in a human body to a specified position.

The camera unit 12 is mounted in the corresponding camera unit mounting hole in the head shell 5; one end of the camera cable 11 is connected with the camera unit 12, and the other end of the camera cable is connected with the camera control device through the camera control and power interface on the connection body 3; and the camera unit 12 is used for photographing and recording information inside the human body, and transmitting the information to the external camera control device through the camera cable 11, so as to realize collection of pictures and videos of the camera unit 12. At the same time, an external power supply supplies power to the camera unit 12 through the camera cable 11.

The three circuit boards are mounted in a mounting slot in the head shell 5, and the three pressure sensors 14 are respectively connected to the corresponding three circuit boards in the head shell. In order to facilitate the installation of the sensor feeler levers 9, three arc-shaped mounting blocks 6 fitted to an outer circumference of the head shell 5 are disposed. Two mounting holes used for mounting the sensor feeler levers 9 are arranged on each arc-shaped mounting block 6. During installation, the sensor feeler levers 9 are mounted on the arc-shaped mounting blocks 6, and sensing ends of the sensor feeler levers 9 protrude out of the arc-shaped mounting blocks 6. Later, the arc-shaped mounting blocks 6 are mounted on an outer circumferential surface of the head shell 5, and the three arc-shaped mounting blocks 6 are uniformly distributed along the circumferential direction of the head shell 5. Each arc-shaped mounting block 6 is located by one locating block 7. The pressure sensors 14 are arranged in a circular arc slot of the head shell 5 to ensure that a sensitive region of each thin-film type pressure sensor 14 is located in a region right below the two sensor feeler levers 9 on the same arc-shaped mounting block 6. After the above installation is completed, a head end cover 4 is mounted on the head shell 5.

One end of the tool channel tube 13 is connected with the tool channel interface on the head shell 5, and the other end is connected with the tool channel interface on the connection body 3 after passing through the circular-ring-shaped magnet 10, the head shell 5 and the channel module 2. The tool channel tube 13 is used for putting in and taking out of biopsy forceps and other tiny surgical instruments and an aspiration operation of an aspirator. For example, when the aspiration operation is required, an aspirator can be externally connected to perform the aspiration operation by using a tool channel.

One end of the water/air supply channel tube 15 is connected with the water/air nozzle via the water/air channel interface on the head shell 5, and the other end is connected with the water supply equipment interface and the air supply equipment interface on the connection body 3 respectively after passing through the circular-ring-shaped magnet 10, the head shell 5 and the channel module 2 in sequence, and then is connected with external water supply equipment and air supply equipment. The water/air nozzle can flush a camera lens in the camera unit 12 to ensure the photographing quality of a camera.

A sealing tube is wrapped outside the camera cable 11, the tool channel tube 13 and the water/air channel tube 15. One end of the sealing tube is connected with the capsule type head 1, and the other end is connected with the connection body 3.

The working principle is as follows:
When the capsule-like robot is used to check a small lumen in the human stomach and intestine, or a small intestine, the capsule type head 1 is inserted into the human body, and the channel module 2 enters the human body along with the capsule type head 1.

After the capsule type head 1 enters the human body, an external magnetic field is used to drive the capsule type head 1 to move. The external magnetic field may be a single magnetic field or a plurality of magnetic fields. When passing through the small lumen or driven by a plurality of magnetic fields, multiple sides of the capsule type head 1 are in contact with the outside, and the sensor feeler levers 9 mounted on the capsule type head 1 would be in contact with the outside and then squeeze the thin-film type pressure sensors 14 mounted in the capsule type head 1. The force induction module processes signals of the thin-film type pressure sensors 14 through the force data output unit, and transmits, in a wireless transmission manner, force data information corresponding to the sensors to an external control system. The external control system processes the received force data information of the sensors, and can determine a deviation between the current posture and the specified path by comparing the magnitudes of the received induction forces of the respective sensors, so as to adjust the current posture to realize force data information-based path planning, and motion control can be better performed. In addition, if the magnitude of the induction force of the sensor on a certain contact side is monitored to exceed a set safety threshold, the external control system makes a response to stop the action or to increase the distance between the external magnetic field at this contact side and the capsule type head to ensure the safety of the human body. The force induction units in the force induction module are symmetrically distributed on the capsule type head 1, and the sensor feeler levers are in discrete distribution to ensure that an interactive contact force between the capsule and the gastrointestinal wall can be detected on the occasion of a tiny lumen and driving by a plurality of external magnetic fields.

The channel module 2 adopts a flexible tube, and the camera cable adopts a highly-flexible filament, so that in the inspection process, no acute pain would be caused to the human body. Due to the presence of the channel module 2, a water/air flushing operation may be performed on the camera lens, or biopsy forceps may be used to perform a sampling operation on the tool channel, and some tiny surgical instruments may also be used to perform a surgical operation. The tool channel may also be used to perform an aspiration operation to discharge excessive water/air and sewage in the stomach and intestine out of the body.

The movement of the capsule type head 1 is realized in a way that the external magnetic field drives the magnet 10 in the capsule type head 1. When the external magnetic field drives the capsule type head 1 to move and reach a suspected lesion part or a set position needing surgical treatment, the capsule type head stops moving, and a tiny surgical instrument puts into the human body through the tool channel tube 13 to complete sampling, sprinkling and other microsurgical operations to finish the inspection.

### Embodiment 2

The present embodiment provides another capsule-like robot having a symmetrical lateral force induction function. Other components and connection relationships are the same as those in Embodiment 1 except the capsule type head 1 and the force induction module.

In the present embodiment, thin-film type pressure sensors 27 in the force induction module are arranged on bosses on a surface of the capsule type head. Compared with Embodiment 1 that the sensitive regions of the single sensors rely on the discrete distribution of the sensor feeler levers, the present embodiment is that four pressure sensors 27 are uniformly distributed along the circumferential direction of the capsule type head.

Referring to Figs. 6 to 8, the capsule type head includes: a circular-ring-shaped magnet 10, a camera unit 12, a circuit board mounting plate 28, and a head shell. The installation modes for the circular-ring-shaped magnet 10 and the camera unit 12 are the same as those in Embodiment 1. In the present embodiment, four bosses used for mounting the thin-film type pressure sensors 27 are uniformly distributed on an external circumferential surface of the head shell 24 along the circumferential direction, and two ends of the bosses are respectively provided with limiting boss fasteners 25, 26 arranged, in a sleeving manner, on the outer circumference of the head shell 24 to axially limit the thin-film type pressure sensors 27.

The force induction module includes: a force induction unit and a force data output unit. Since the thin-film type pressure sensors 27 in the present embodiment are directly mounted on the outer surface of the capsule type head to be in contact with the outside, the force induction unit in the present embodiment does not include sensor feeler levers. The force data output unit includes four circuit boards. The four circuit boards are respectively a circuit board A29, a circuit board B30, a circuit board C31 and a circuit board D32. The four circuit boards are uniformly distributed in a spacing manner along a circumferential direction of the head shell 24, so as to reduce the occupied volume as much as possible. A circuit board mounting plate 28 used for mounting the four circuit boards are arranged inside the head shell 24. The circuit board A29 is integrated with a battery 20, the circuit board B30 is integrated with an operational amplifier circuit 22, the circuit board C31 is integrated with a wireless transmission module 21, and the circuit board D32 is integrated with an analog-to-digital conversion module 23. Each thin-film type pressure sensor 27 is connected with the circuit board opposite to it. Power supplying and signal transmission are performed between the circuit boards through cables. Therefore, after signals collected by the four thin-film type pressure sensors 27 are processed by the operational amplifier circuit 22 and the analog-to-digital conversion module 23, the signals are finally transmitted in a wireless transmission manner via the wireless transmission module 21 to external control equipment.

### Embodiment 3

The present embodiment provides another capsule-like robot having a symmetrical lateral force induction function. Other components and connection relationships therebetween are the same as those in Embodiment 1 except the capsule type head 1 and the force induction module.

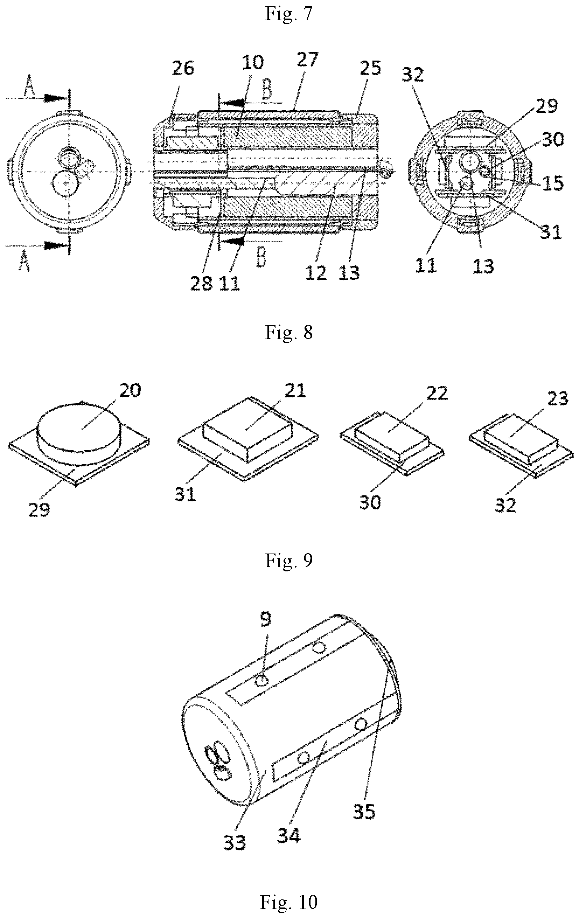

As shown in Figs. 10 to 12, in the present embodiment, a cubic magnet 36 which is hollow inside is used. Therefore, the capsule type head 1 includes: a camera unit 12, a cubic magnet 36, and a head shell 33. The cubic magnet 36 is mounted in a rectangular slot in the head shell 33, and the camera unit 12 is mounted in a mounting hole in the head shell 33. A water/air channel hole, a tool channel hole and a camera cable channel hole are machined inside the head shell 33; in order to reduce the volume of the capsule type head 1 as much as possible, the water/air channel hole, the tool channel hole and the camera cable channel hole all fall within an envelope area for the rectangular slot used for mounting the cubic magnet 36, that is, the water/air channel hole, the tool channel hole and the camera cable channel hole run through the rectangular slot of the cubic magnet 36. Therefore, after passing through the center hole of the cubic magnet 36 and the water/air channel hole in sequence, the water/air channel tube 15 communicates with the water/air nozzle. After passing through the center hole of the cubic magnet 36 and the camera cable channel hole in sequence, the camera cable 11 is connected with the camera unit 12 mounted in the camera unit mounting hole.

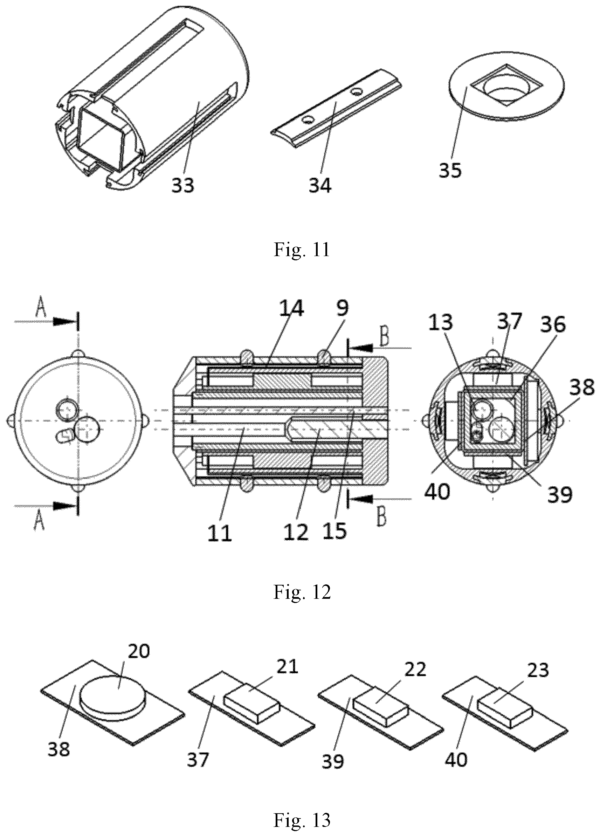

The force induction module includes: a force induction unit and a force data output unit. The force induction unit in the present embodiment includes eight sensor feeler levers 9 and four thin-film type pressure sensors. Each thin-film type pressure sensor responds to two sensor feeler levers 9, and the sensor feeler levers 9 are used for sensing a pressure and transmitting the sensed pressure to the pressure sensor. In the present embodiment, the installation modes of the sensor feeler levers and the thin-film type pressure sensors on the head shell are the same as those in Embodiment 1, and the induction principle is completely the same as that in Embodiment 1. The four circuit boards are respectively integrated with a wireless transmission module 21, a battery 20, an operational amplifier circuit 22, and an analog-to-digital conversion module 23, as shown in Fig. 13. Furthermore, the four circuit boards are uniformly distributed in a spacing manner along the circumferential direction of the head shell 33, so as to reduce the occupied volume as much as possible.

### Embodiment 4

The present embodiment provides another capsule-like robot having a symmetrical lateral force induction function. Other components and connection relationships therebetween are the same as those in Embodiment 2 except the force induction module.

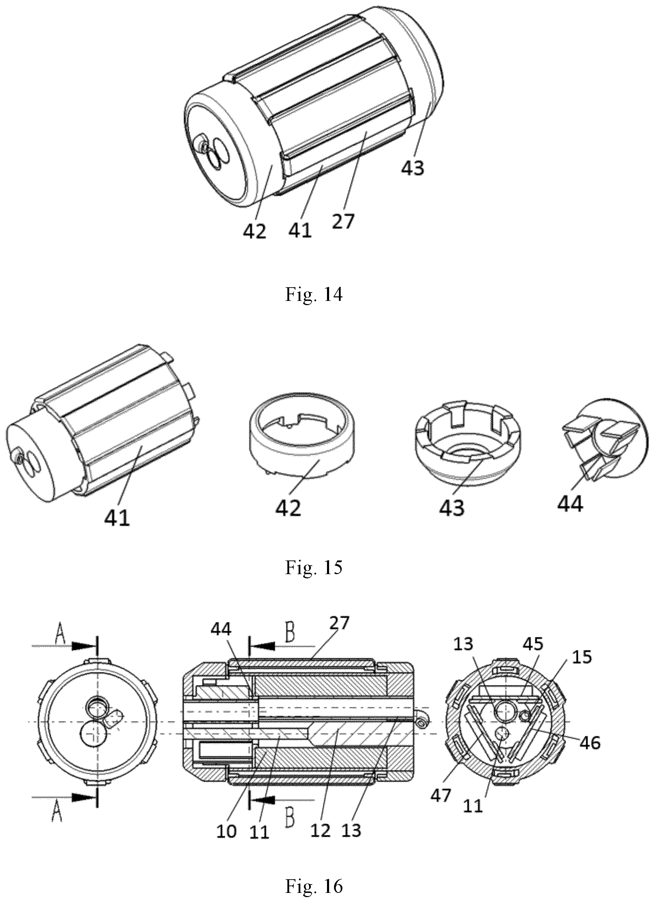

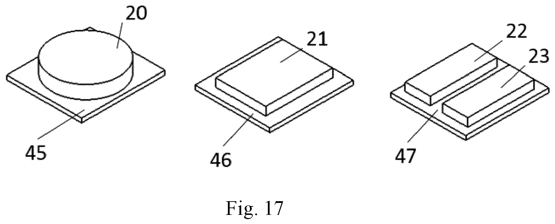

Referring to Figs. 14 to 17, the capsule-like robot differs from Embodiment 2 in that there are six thin-film type pressure sensors 14 in the force induction module, and the force data output unit in the force induction module includes three circuit boards. Each circuit board corresponds to two thin-film type pressure sensors 14.

The six thin-film type pressure sensors 14 are directly mounted on the outer surface of the capsule type head and are in contact with the outside. That is, in the present embodiment, six bosses used for mounting the thin-film type pressure sensors 14 are uniformly distributed on an external circumferential surface of the head shell 41 along the circumferential direction, and two ends of the bosses are respectively provided with limiting boss fasteners 42, 43 arranged, in a sleeving manner, on the outer circumference of the head shell 41 to axially limit the thin-film type pressure sensors 14.

A circuit board mounting plate 44 used for mounting the three circuit boards is arranged inside the head shell 41. The three circuit boards are mounted inside the head shell 41 through the circuit board mounting plate 44, and are uniformly distributed along the circumferential direction of the head shell 41. The three circuit boards are respectively a circuit board A45, a circuit board B46, and a circuit board C47. The circuit board A45 is integrated with a battery 20, the circuit board B46 is integrated with a wireless transmission module 21, and the circuit board C47 is integrated with an operational amplifier circuit 22 and an analog-to-digital conversion module 23. Power supplying and signal transmission are performed between the circuit boards through cables. Two thin-film type pressure sensors 14 form one group, and are connected with the corresponding circuit board, so that after signals collected by the six pressure sensors 14 are processed by the operational amplifier circuit 22 and the analog-to-digital conversion module 23, the signals are finally transmitted in a wireless transmission manner via the wireless transmission module 21 to external control equipment.

## Claims

1. A capsule-like robot having a symmetrical lateral force induction function, **characterized by** comprising a capsule type head (1), a connection body (3), a force induction module arranged in the capsule type head (1), and a channel module (2) used for connecting the capsule type head (1) with the connection body (3),
wherein the capsule type head (1) comprises: a head shell (5), a magnet mounted inside the head shell (5), and a camera module (12) integrated with a light source; the magnet performs location and motion control on the capsule type head (1) under the driving of an external magnetic field; a magnet mounting slot, a tool channel hole and a camera cable channel hole are machined in the head shell (5); an end, opposite to an end connected with the channel module (2), of the head shell (5) is provided with a mounting slot which communicates with the camera cable channel hole and is used for mounting the camera module (12);
the force induction module is used for sensing pressure data of each contact side under a condition that the capsule-like robot is in multilateral symmetrical contact with an external environment; the force induction module comprises: a force induction unit and a force data output unit; the force induction unit comprises two or more thin-film type pressure sensors (14) uniformly distributed in a spacing manner along a circumferential direction of the head shell (5); the pressure sensors (14) are used for sensing pressure data of the external environment on a mounting side of the capsule type head (1), and transmitting the sensed pressure data to the force data output unit; and the force data output unit performs signal conditioning and analog-to-digital conversion on the received pressure data, and transmits the data to an external upper computer in a wireless manner; and
the channel module (2) comprises: an external sealing tube, and a camera cable (11) and a tool channel tube (13) which are wrapped in the external sealing tube;
a tool channel interface (3-3) and a camera control and power interface (3-4) are arranged on the connection body (3); the tool channel interface (3-3) communicates with the tool channel tube (13) in the channel module (2); one end of the camera cable (11) is connected with the camera module (12) through the camera cable channel inside the head shell (5), and the other end of the camera cable is connected with an external power supply and a camera control device through the camera control and power interface (3-4) on the connection body (3).

2. The capsule-like robot having the symmetrical lateral force induction function according to claim 1, **characterized in that** the channel module (2) further comprises a water/air channel tube (15) wrapped in the external sealing tube; a water/air channel hole is machined inside the head shell (5); the end, opposite to an end connected with the channel module (2), of the head shell (5) is provided with a water/air nozzle communicating with the water/air channel tube (15); a water supply equipment interface (3-1) and an air supply equipment interface (3-2) are arranged on the connection body (3); one end of the water/air channel tube (15) communicates with the water/air nozzle through the water/air channel hole, and the other end of the water/air channel tube communicates with the water supply equipment interface (3-1) and the air supply equipment interface (3-2).

3. The capsule-like robot having the symmetrical lateral force induction function according to claim 1, **characterized in that** after obtaining the pressure data of the external environment on each contact side of the capsule type head (1), the upper computer takes the pressure data as a contact interaction force between the external environment and the capsule type head to perform safety monitoring on the capsule-like robot, specifically as follows: a safety threshold of the contact interaction force of each contact side is set in the upper computer; and when the pressure data sensed by the pressure sensor of a certain contact side exceeds the corresponding safety threshold, the capsule-like robot is controlled to stop moving or a distance between the external magnetic field and the capsule type head (1) is increased.

4. The capsule-like robot having the symmetrical lateral force induction function according to claim 1, 2 or 3, **characterized in that** in the force induction unit, the thin-film type pressure sensors are arranged inside the head shell; sensor feeler levers mounted at an outer circumference of the head shell and having induction ends protruding from an outer surface of the head shell sense a pressure and transmit the sensed pressure to the thin-film type pressure sensors; and each thin-film type pressure sensor corresponds to one or more sensor feeler levers.

5. The capsule-like robot having the symmetrical lateral force induction function according to claim 4, **characterized in that** an installation mode of the sensor feeler levers is as follows: a same number of arc-shaped mounting blocks as the thin-film type pressure sensors are arranged on an outer circumferential surface of the head shell; one or more mounting holes for the sensor feeler levers are arranged on each arc-shaped mounting block along an axial direction; induction ends of the sensor feeler levers protrude from the arc-shaped mounting blocks; the arc-shaped mounting blocks are uniformly distributed along the circumferential direction of the head shell; the thin-film type pressure sensors are arranged inside the head shell; and a sensitive region of each pressure sensor is located in a region right below the sensor feeler levers on the same arc-shaped mounting block.

6. The capsule-like robot having the symmetrical lateral force induction function according to claim 1, 2 or 3, **characterized in that** in the force induction unit, the sensitive regions of the thin-film type pressure sensors are directly arranged on bosses on the outer surface of the head shell.

7. The capsule-like robot having the symmetrical lateral force induction function according to claim 2, **characterized in that** the magnet is of a hollow structure; the water/air channel hole, the tool channel hole and the camera cable channel hole all fall within an enveloping range corresponding to a center hole of the magnet, that is, the water/air channel hole, the tool channel hole and the camera cable channel hole respectively run through the center hole of the magnet.

8. The capsule-like robot having the symmetrical lateral force induction function according to claim 2, **characterized in that** the external sealing tube, the camera cable (11), the tool channel tube (13), and the water/air channel tube (15) in the channel module (2) are all flexible tubes.

9. The capsule-like robot having the symmetrical lateral force induction function according to claim 1, 2 or 3, **characterized in that** the force data output unit comprises: a wireless transmission module, an operational amplifier circuit module, an analog-to-digital conversion module, and a battery used for supplying power to the above three modules; the wireless transmission module, the operational amplifier circuit module, the analog-to-digital conversion module, and the battery are respectively integrated on one or more circuit boards; the circuit boards are uniformly distributed in a spacing manner along the circumferential direction of the head shell (5); and the circuit boards are connected through cables, so as to perform power supplying and signal transmission.

10. The capsule-like robot having the symmetrical lateral force induction function according to claim 9, **characterized in that** two or more of the thin-film type pressure sensors (14) are respectively connected with the circuit boards close to the thin-film type pressure sensors.
